Europäisches Patentamt

European Patent Office

Office européen des brevets

(11) Publication number: **0 263 655**
**A2**

# (12) EUROPEAN PATENT APPLICATION

(21) Application number: 87308776.1

(22) Date of filing: 02.10.87

(51) Int. Cl.⁴: **C07K 7/00 , A61K 37/02**

(30) Priority: 03.10.86 JP 236658/86

(43) Date of publication of application:
**13.04.88 Bulletin 88/15**

(84) Designated Contracting States:
**DE ES FR GB IT**

(71) Applicant: **Dainippon Pharmaceutical Co., Ltd.**
**25, Doshomachi 3-chome Higashi-ku**
**Osaka-shi, Osaka 541(JP)**

Applicant: **AJINOMOTO CO., INC.**
**5-8, Kyobashi 1-chome, Chuo-ku**
**Tokyo 104(JP)**

(72) Inventor: **Nakamura, Hideo**
**449-65, Oji-cho**
**Tenri-shi Nara-ken(JP)**
Inventor: **Ishii, Katsumi**
**12-1-308, Kojogaoka**
**Otsu-shi Siga-ken(JP)**
Inventor: **Ariyoshi, Yasuo**
**1-1, Suzuki-cho Kawasaki-ku**
**Kawasaki-shi Kanagawa-ken(JP)**
Inventor: **Nio, Noriki**
**1-1, Suzuki-cho Kawasaki-ku**
**Kawasaki-shi Kanagawa-ken(JP)**

(74) Representative: **Ford, Michael Frederick et al**
**MEWBURN ELLIS & CO. 2/3 Cursitor Street**
**London EC4A 1BQ(GB)**

(54) **Novel peptides having antiallergic activity.**

(57) Peptides selected from peptides having 1st to 20th, 1st to 29th, or 17th to 28th amino acid sequence in the formula [I]:

Ala-Asp-Ser-Asn-Pro-Arg-Gly-Val-Ser-Ala-
Tyr-Leu-Ser-Arg-Pro-Ser-Pro-Phe-Asp-Leu-
Phe-Ile-Arg-Lys-Ser-Pro-Thr-Ile-Thr

and peptides having 1st to 21st, 3rd to 47th, 10th to 17th, 10th to 24th, 22nd to 40th, 38th to 50th, 39th to 45th, 40th to 45th, or 40th to 54th amino acid sequence in the formula [II]:

Gln-X -Arg-Val-Thr-His-Pro-His-Leu-Pro-
Arg-Ala-Leu-Met-Arg-Ser-Thr-Thr-Lys-Thr-
Ser-Gly-Pro-Arg-Ala-Ala-Pro-Glu-Val-Tyr-
Ala-Phe-Ala-Thr-Pro-Glu-Trp-Pro-Gly-Ser-
Arg-Asp-Lys-Arg-Thr-Leu-Ala-X -Leu-Ile-
Gln-Asn-Phe-Met

wherein X is Cys or Ser,

and partially modified peptides at an amino acid residue thereof, and salts thereof, have excellent anti-allergic activity and are useful for the prophylaxis and treatment of allergic diseases.

## NOVEL PEPTIDES HAVING ANTIALLERGIC ACTIVITY

This invention relates to novel peptides and partially modified compounds at the amino acid residue(s) thereof which have a part of the amino acid sequences of immunoglobulin E (abbreviated as "IgE"). The peptides of this invention have excellent antiallergic activity and are useful for prophylaxis and treatment of allergic diseases.

Technical Background and Prior Art

The living bodies prevent themselves from nonself by recognizing the nonself and removing it. That is, they recognize a part of foreign material as an antigen and produce an antibody against the antigen. The produced antibody reacts with an antigen which invades newly into the living bodies to form an antigen-antibody complex and thereby the foreign material is removed. The antigen-antibody reaction affects occasionally on the living bodies to induce injury of their tissues, which penomenon is called as "allergy". The allergic reaction has been classified into four types (I to IV) based on the mechanism by P.H. Gell and R.R.A. Coombs. The types I through III of allergy are based on the reaction of an antigen and a humoral antibody, which occur within a very short time and hence are called as "immediate allergy", and the type IV of allergy is based on cellular immunity, which occurs comparatively slowly and hence is called as "delayed allergy". Among these allergies, the type I of allergy is the representative one and includes various diseases such as bronchial asthma, allergic rhinitis, food allergy (e.g. urticaria, gastrointestinal allergy, etc.) and the like. These allergic diseases have recently increased and become one of social problems.

The allergic reaction in the type I is basically induced by binding an immunoglobulin E (IgE) antibody against an allergen which is a substance capable of inducing allergy with an IgE receptor (called also as "Fcε receptor") which exists on the surface of mastocyte in tissue or of basocyte in blood. That is, when an allergen invades into the living body, an IgE antibody against it is produced in B-lymphocytes. On the other hand, there are several hundred thousand IgE receptors on the surface of the mastocyte or basocyte, and the IgE antibody binds to the IgE receptor with high affinity. To the IgE bound onto the cell surface binds further a polyvalent allergen which invades again, and thereby some IgE molecules are crosslinked. This induces a certain mutual response between receptor molecules and thereby enzymes in cell membrane are activated. As the result, the granules existent within the mastocytes and basocytes are removed from the cells, and said degranulation phenomenon being accompanied with release of chemical mediators (e.g. histamine, SRS-A, serotonin, etc.) existent within the granules, which induce increased vascular permeability, contraction of smooth muscle, increased secretion, and the like, and further induce various allergic diseases.

There have usually been used various antiallergic drugs, such as an antagonist to the chemical mediators (e.g. antihistaminics, etc.), an agent for inhibiting release of the chemical mediators (e.g. disodium cromoglycate, etc.), steroids, and the like. However, these knowns drugs do not inhibit the binding between the IgE - Fcε receptor which is the basic reaction in the type I allergy and further use of these known drugs is restricted because of the undesirable side effect and also limited administration method.

In view of the mechanism of the type I allergy, unless the IgE binds to the Fcε receptor on the surface of mastocytes or basocytes, the type I allergy will not be induced even by invasion of allergen. Accordingly, if the binding of the IgE to Fcε receptor can be inhibited, degranulation does not occur, and hence, the allergic diseases can be prevented and remedied.

Since the IgE molecule binds to IgE receptor, there must exist a site of binding to the receptor anywhere in the IgE molecule. If a substance contains a site of binding with the receptor in IgE, the substance will bind to the Fcε receptor in competition with IgE, which results in inhibition of the antigen-antibody reaction and thereby it shows an antiallergic activity. If such a substance is found, it will become a new type of antiallergic agent.

Based upon the above idea, the following various approaches have been tried.

(1) Inhibition of Prausnitz-Kuestner reaction by Fcε fragment (a peptide fragment containing $C_\epsilon 2$-$C_\epsilon 4$ domain) which is obtained by partial hydrolysis of IgE as reported by Stanworth [cf. Stanworth, D.R., Nature, 233, 310 (1970)]

(2) Cε2-Cε4 domain being capable of binding to human basocytes and exhibiting antiallergic activity, said Cε2-Cε4 domain being prepared by inserting cDNA encoding from Cε2 to Cε4 into Escherichia coli and then expressing it therefrom [cf. Liu, F. et al., Proc. Ntl. Acad. Sci. USA, 81, 5369 (1984); Kenten, J. et al., Proc. Natl. Acad. Sci. USA, 81, 2955 (1984); Geha, R.S. et al., Nature, 315, 577 (1985); and Coleman, J.W. et al., Eur. J Immunol., 15, 966 (1985)]

However, in these reports, it fails to specify the antiallergic site in IgE, i.e. the site of binding of the IgE receptor.

Besides, Dorrington and Bennich have suggested that the receptor-binding site exists on Cε3-Cε4 domain from the observation of the change in the secondary structure of IgE after gently heating with a circular dichroism [cf. Dorrington, K.J. and Bennich, H.H., Immunological Rev., 41, 3 (1978)].

Japanese Patent Second Publication (Kokoku) No. 2318/1985 discloses peptides having an amino acid sequence of Asp-Pro-Arg, Ser-Asp-Pro-Arg, Asp-Ser-Asp-Pro-Arg or Ala-Asp-Ser-Asp-Pro-Arg, but these peptides have different amino acid sequence from that of the Fc moiety of human IgE. It is also reported that a peptide having an amino acid sequence corresponding to that of the Fc moiety of human IgE does not show any antiallergic activity [cf. Bennich, H. et al., Int. Arch. Allergy Appl. Immunol., 53, 459-468 (1977)].

Japanese Patent Publication (PCT) No. 500025/1987 discloses a peptide fragment which exists on Cε3-Cε4 domain, but the peptide of the present invention is clearly distinguished from the peptide disclosed in said Japanese patent publication.


Summary Description of the Invention

The present inventors have prepared various peptides containing a part of amino acid sequences in the Fc moiety of human IgE and have studied the antiallergic activity of these peptides, and have found that all peptides of those do not show antiallergic activity but a certain specific peptides having a specific amino acid sequence can show the desired antiallergic activity.

An object of the invention is to provide a novel peptide having excellent antiallergic activity. Another object of the invention is to provide a pharmaceutical composition for the prophylaxis and treatment of allergic diseases comprising as an active ingredient said peptide. These and other objects and advantages of the invention will be apparent from the following description.


Detailed Description of the Invention

The present invention provides (1) a peptide selected from the group consisting of a peptide having 1st to 20th amino acid sequence, a peptide having 1st to 29th amino acid sequence, and a peptide having 17th to 28th amino acid sequence in the formula [1]:

Ala-Asp-Ser-Asn-Pro-Arg-Gly-Val-Ser-Ala   -
1                                          10

Tyr-Leu-Ser-Arg-Pro-Ser-Pro-Phe-Asp-Leu   -
                                       20

Phe-Ile-Arg-Lys-Ser-Pro-Thr-Ile-Thr
                                29

or (2) a peptide selected from the group consisting of a peptide having 1st to 21st amino acid sequence, a peptide having 3rd to 47th amino acid sequence, a peptide having 10th to 17th amino acid sequence, a peptide having 10th to 24th amino acid sequence, a peptide having 22nd to 40th amino acid sequence, a peptide having 38th to 50th amino acid sequence, a peptide having 39th to 45th amino acid sequence, a peptide having 40th to 45th amino acid sequence, and a peptide having 40th to 54th amino acid sequence in the formula [II]:

Gln-X -Arg-Val-Thr-His-Pro-His-Leu-Pro   -
1                                       10

Arg-Ala-Leu-Met-Arg-Ser-Thr-Thr-Lys-Thr   -
                                       20

Ser-Gly-Pro-Arg-Ala-Ala-Pro-Glu-Val-Tyr   -
                                       30

Ala-Phe-Ala-Thr-Pro-Glu-Trp-Pro-Gly-Ser   -
                                       40

Arg-Asp-Lys-Arg-Thr-Leu-Ala-X -Leu-Ile
50

Gln-Asn-Phe-Met
54

wherein X is Cys or Ser,
or (3) a peptide (1) or (2) partially modified at an amino acid residue thereof, or (4) a salt of any of the peptides (1),(2) and (3).

The above mentioned peptide (1) or (2) may be partially modified (peptide (3) above) at an amino acid residue thereof and yet still be capable of exhibiting antiallergic activity.

Such antiallergic activity may be realized if the peptide is capable of binding to a receptor for Ig E.

The peptides having amino acid sequences of the formula [I] or [II] correspond to peptides having amino acid sequences of 329th to 357th and 417th to 470th in the Fc moiety of human IgE, respectively, or a peptide having the amino acid sequences wherein Cys is replaced with Ser.

The peptide of this invention can be prepared by a conventional liquid phase method or solid phase method. For instance, one starting material with a free carboxyl group corresponding to one of two fragmens, which is divided at an optional site of said peptide, is condensed with other starting material with a free amino group corresponding to other of the fragments by a conventional DCC method (using N,N'-dicyclohexylcarbodiimide), active ester method, and the like. When the functional group(s) of the resultant is (are) protected, it (they) is (are) deprotected.

Any functional group which does not participate in the reaction is usually protected by a protecting group. An amino-protecting group includes, for example, benzyloxy-carbonyl, t-butyloxycarbonyl, p-biphenylisomethyloxy-carbonyl, 9-fluorenylmethyloxycarbonyl, and the like. A carboxy-protecting group includes groups which can form an alkyl ester, benzyl ester, or the like. When the peptide is prepared by a solid phase method, the carboxyl group at the C-terminus is bound to a support, such as a chloromethyl resin, a hydroxymethyl resin, a p-alkoxybenzyl alchohol resin, and the like.

The condensation reaction is usually carried out in the presence of a condensing agent such as dicyclohexyl-carbodiimide, or by using an N-protected active ester or a peptide active ester.

After the condensation reaction is completed, the protecting group (if any) is deprotected by a conventional method. In case of a solid phase method, the C-terminus of the peptide is further cleaved from the resin.

The peptide of this invention thus prepared can be purified by a conventonal purification method, such as ion exchange chromatography, reverse phase high performance liquid chromatography, affinity chromatography, and the like.

The peptide of this invention can be converted into various salts thereof by treating it with various inorganic or organic acids. The salts include inorganic acid salts, such as hydrochloride, hydrobromide, hydroiodide, sulfate, phasphate, etc., and organic acid salts, such as acetate, trifluoroacetate, citrate, maleate, fumerate, tartrate, lactate, methanesulfonate, p-toluenesulfonate, etc. The peptide can also be converted into other salts by treating with an alkali metal or alkaline earth metal hydroxide or with an organic base, for example, potassium salt, sodium salt, calcium salt, lysine salt, etc.

Activities

The peptides of this invention or salts thereof have excellent antiallergic activities and are useful for the prophylaxis and treatment of allergic diseases.

The activities of the peptides of this invention are illustrated by the following experiment. [Inhibitory effect on passive cutaneous anaphylaxis]:

This test was carried out by the method of Perper et al. [cf. J. Pharmacol. Exp. Ther., 193, 594 (1975)] with minor modifications.

Male Wistar rats (140 to 200 g) were injected with 0.1 ml of a dilute solution of mouse antiserum to egg albumin prepared by a similar method to that of Levine et al. [cf. Levine, B.B. and Vaz, N.M., Int. Arch. Allergy Appl. Immunol., 39, 156-168 (1970)] in two sites of the shaved dorsal skin. Forty-eight hours later each rat was challenged by an intravenous injection of 2 mg of the antigen together with 1 ml of a 0.5 % Evan's blue solution. The rats were sacrificed 30 minutes after the challenge with carbon dioxide gas. The dorsal skin was removed and the area of the blueing lesions was measured. The average value of the two lesions was regarded as the response of the rat. The test compounds, dissolved or suspended in a physiological saline solution, were administered into the skin at the responded lesion three times 24 hours and 3 hours before the antiserum challenge and at the same time as the antiserum challenge in a dose of 10 μg, 30 μg or 100 μg/site.

The inhibitory rate was determined by comparing the responses of the rats given each test compound with those of the rats given only the physiological saline solution. The results are shown in Table 1. The inhibitory rate in the table is shown by the average of 5 rats in each group.

Table 1

| Test compound | Dose (μg) | Inhibitory rate (%) |
|---|---|---|
| 1 | 100 | 32.4* |
| 2 | 100 | 32.9** |
| 3 | 100 | 33.2** |
| 4 | 30 | 20.1* |
| 5 | 100 | 23.2* |
| 6 | 100 | 15.9* |
| 7 | 100 | 20.0* |
| 8 | 100 | 25.5* |
| 9 | 100 | 25.4* |
| 10 | 100 | 28.3** |
| 11 | 10 | 16.9* |
| 12 | 100 | 25.2** |
| Ref. Compound | | |
| 13 | 100 | -3.9 |
| 14 | 100 | -3.8 |

[Notes]: *) $0.01 < P < 0.05$ and **) $p < 0.01$, significantly different from the vehicle control.

The test compounds 1 to 12 correspond to the compounds in Examples 1 to 12, respectively.

The Reference Compounds 13 and 14 are the compounds as shown in Reference Example.

As is clear from the above experiment, the peptides and their salt of this invention show excellent antiallergic activity and are useful as an antiallergic agent for the prophylaxis and treatment of various allergic diseases, such as bronchial asthma, allergic rhinitis, urticaria, atopic dermatitis, eczema, allergic ophthalmia, and the like.

The peptides and their salts of this invention can be administered by oral, parenteral, intrarectal or topical route, preferably by topical route. The dose of the peptides and their salts may very depending on the kinds of compounds, administration methods, age and sex of the patients, severity of the diseases, and the like, but is usually in the range of 0.001 to 1000 mg, preferably 0.01 to 10 mg. at one time, and the compounds are administered one to three times per day in the above dose.

5

The peptides and their salts are usually used in the form of a conventional pharmaceutical preparation in admixture with conventional pharmaceutically acceptable carriers or diluents. The carriers and diluents include any conventional ones which are usually used for preparing pharmaceutical preparations and do not react with the peptides or their salts. Suitable examples of the carriers asnd diluents are lactose, glucose, mannitol, dextrin, cyclo-dextrin, starches, white suger, magnesium metasilicate aluminate, synthetic aluminum silicate, crystalline cellulose, sodium carboxymethyl cellulose, hydroxypropyl starch, calcium carboxymethyl cellulose, ion exchange resin, methyl cellulose, gelatin, gum arabic, hydroxypropyl cellulose, low substitution degree hydroxypropyl cellulose, hydroxypropyl methyl cellulose, polyvinylpyrrolidone, polyvinyl alcohol, light silicic acid anhydride, magnesium stearate, talc, tragacant, bentonite, bee gum, carboxyvinyl polymer, titanium oxide, sorbitan fatty acid esters, sodium laurylsulfate, glycerin, glycerin fatty acid esters, purified lanolin, glycerogelatin, polysolvate, macrogol, vegetable oils, waxes, liquid paraffin, white petroleum, fluorocarbon, nonionic surfactants, propylene glycol, water, and the like.

The pharmaceutical preparation includes tablets, capsules, granules, powders, syrups, suspensions, suppositories, ointments, creams, gels, adhesive preparations, inhalants, injections, and the like. These preparations can be prepared by a conventional method. The liquid preparations may be in the form which is dissolved or suspended in an appropriate solvent such as water or others when used. The tablets and granules may be coated with a conventional coating material in a usual manner. The injection is usually prepared by dissolving the peptide or its salt of the invention in water, but occasionally dissolving in physiological saline solution or glucose solution, to which a buffering agent and perservative may optionally be incorporated.

The pharmaceutical preparation of this invention contains the peptide or its salt of the invention in an amount of 0.2 % by weight or more, preferably 0.5 to 70 % by weight, based on the whole weight of the preparation. The preparation may also contain other therapeutically effective ingredients.

This invention is illustrated by the following Examples but should not be construed to be limited thereto.

The abbreviations used in the specification mean as follows.

Ala: Alanine
Arg: Arginine
Asn: Asparagine
Asp: Aspartic acid
Cys: Cysteine
Gln: Glutamine
Glu: Glutamic Acid
Gly: Glycine
His: Histidine
Ile: Isoleucine
Leu: Leucine
Lys: Lysine
Met: Methionine
Phe: Phenylalanine
Pro: Proline
Ser: Serine
Thr: Threonine
Trp: Tryptophane
Tyr: Tyrosine
Val: Valine
Fmoc: 9-Fluorenylmethyloxycarbonyl
tBu: t-Butyl
DMF: Dimethylformamide
Mtr: 4-Methoxy-2,3,6-trimethylbenzenesulfonyl
Boc: t-Butyloxycarbonyl
DOD: Dimethoxydithyl
DMF: Dimethylformamide
ONp: p-Nitrophenyl ester
HOBt: 1-Hydroxybenzotriazole
All amino acids are in the L-form.


Example 1

6

Preparation of Ala-Asp-Ser-Asn-Pro-Arg-Gly-Val-Ser-Ala-Tyr-Leu-Ser-Arg-Pro-Ser-Pro-Phe-Asp-Leu-Phe-Ile-Arg-Lys-Ser-Pro-Thr-Ile-Thr (Compound 1):

Fmoc-Thr(tBu)-resin (i.e. p-alkoxybenzyl alcohol resin introduced with Fmoc-Thr(tBu) in an amount of 0.45 mmol/g of resin) (666 mg) was transfered to a Merrifield solid phase reaction vessel equipped for agitation, and is suspended in dimethylformamide (20 ml) and shaken for 30 minutes, by which Fmoc-Thr-(tBu)-resin was swollen.

The resultant was subjected to the following Fmoc group-deprotecting cycle.

(a) Shaking in DMF (20 ml) for one minutes, followed by filtering (twice),

(b) Shaking in 20 % piperidine-DMF solution (20 ml) for three minutes, followed by filtering,

(c) Shaking in 20 % piperidine-DMF solution (20 ml) for 10 minutes, followed by filtering,

(d) Washing twice with DMF (20 ml),

(e) Washing twice with isopropanol (20 ml).

At this stage, it was confirmed by Kaiser method [cf. E. Kaiser et al., Anal. Biochem., 34 , 595 (1970)] whether Fmoc group was completely deprotected or not. If it was incompleted, the above deprotecting cycle was repeated. If it was completely deprotected, the resultant was subjected to the following synthetic cycle.

(f) Swelling the H-Thr(tBu)-resin obtained by the above Fmoc group-deprotecting cycle by shaking twice in DMF (20 ml), by which the resin was swollen,

(g) Adding thereto a solution of Fmoc-Ile (318 mg, 0.9 mmole) and HOBt (146 mg, 1.08 mmole) in DMF (20 ml) and shaking the mixture for one minute,

(h) Adding thereto a solution of 1M dicyclohexyl-carbodiimide in methylene chloride (0.99 ml) and shaking the mixture for 70 minutes,

(i) Washing twice with DMF (20 ml),

(j) Washing twice with isopropanol (20 ml).

At this stage, it was confirmed by Kaiser method whether the condensation reaction was completed or not. If it was incompleted, the above condensation cycle was repeated. If it was completed, the reaction product was subjected again to Fmoc group-deprotecting cycle and then to the condensation cycle wherein the step (g) was carried out by using Fmoc-Thr(tBu). Thereafter, by repeating the Fmoc group-deprotecting cycle and Fmoc-amino acid condensation cycle likewise, there were condensed with Fmoc-Pro. Fmoc-ser-(tBu), Fmoc-Lys(Boc), Fmoc-Arg(Mtr), Fmoc-Ile, Fmoc-Phe, Fmoc-Leu, Fmoc-Asp(OtBu), Fmoc-Phe, Fmo-Pro, Fmoc-Ser(tBu), Fmoc-Pro, Fmoc-Arg(Mtr), Fmoc-Ser(tBu), Fmoc-Leu, Fmoc-Tyr(tBu), Fmoc-Ala, Fmoc-Ser(tBu), Fmoc-Val, Fmoc-Gly, Fmoc-Arg(Mtr), Fmoc-Pro, Fmoc-Asn(DOD), Fmoc-Ser(tBu), Fmoc-Asp-(OtBu), and Boc-Ala.

The thus obtained Boc-Ala-Asp(OtBu)-Ser(tBu)-Asn(DOD)-Pro-Arg(Mtr)-Gly-Val-Ser(tBu)-Ala-Tyr(tBu)-Leu-Ser(tBu)-Arg(Mtr)-Pro-Ser(tBu)-Pro-Phe-Asp(OtBu)-Leu-Phe-Ile-Arg(Mtr)-Lys(Boc)-Ser(tBu)-Pro-Thr(tBu)-Ile-Thr(tBu)-resin was subjected to the cleavage of resin.

That is, the product was washed twice with methylene chloride (20 ml) and suspended in a mixture of methylene chloride (10 ml) - anisole (2.00 ml) - thiophenol (0.66 ml), and thereto was added trifluoroacetic acid (20 ml) - methylene chloride (2.32 ml), and the mixture was shaken for one hour. After filtering off the resin, ether was added to the filtrate, and the mixture was filtered to give white precipitate (1.02 g). To the product was added a mixture of trifluoroacetic acid (13.5 ml) - thionisole (1.5 ml) - thiophenol (0.60 ml), and the mixture was stirred for 5 hours. To the reaction mixture was added ether, and the mixture was filtered to give white precipitate (1.15 g). The white powder was subjected to a reverse phase high performance liquid chromatography and there was taken a fraction containing the desired Ala-Asp-Ser-Asn-Pro-Arg-Gly-Val-Ser-Ala-Tyr-Leu-Ser-Arg-Pro-Ser-Pro-Phe-Asp-Leu-Phe-Ile-Arg-Lys-Ser-Pro-Thr-Ile-Thr, and the eluted fraction was evaporated to dryness. The residue was dissolved in distilled water and then the mixture was concentrated to dryness. This procedure was repeated several times. Thereafter, a small amount of distilled water was added to the residue and the mixuture was lyophilized to give purified Ala-Asp-Ser-Asn-Pro-Arg-Gly-Val-Ser-Ala-Tyr-Leu-Ser-Arg-Pro-Ser-Pro-Phe-Asp-Leu-Phe-Ile-Arg-Lys-Ser-Pro-Thr-Ile-Thr) 139 mg).

A part of the purified product was taken and subjected to the amino acid sequence analysis with a gas phase protein sequencer. As a result, it had the sequence of Ala, Asp, Ser Asn, Pro, Arg, Gly, Val, Ser, Ala, Tyr, Leu, Ser, Arg, Pro, Ser, Pro, Phe, Asp, Leu, Phe, Ile, Arg, Lys, Ser, Pro, Thr, Ile, Thr in this order, which were identical with the peptide sequence. Besides, the molecular weight of the product was measured with an FAB mass spectromoter, and the obtained data: m/z 3192 (MH+) were well agreed to the theoretical amount. Moreover, the product was hydrolyzed with 6N HCl and subjected to amino acid analysis. As a result, it showed amino acid ratios of Asp. 3.03, Thr 1.99, Ser 4.85, Pro 4.00, Gly 1.05, Ala 1.88, Val 1.12, Ile 1.78, Leu 2.09, Tyr 0.98, Phe 2.03, Lys 1.07, and Arg 1.94, which were well agreed to the theoretical ratios, too. Thus, it was confirmed that the desired peptide was obtained.

The high purity of the product was also confirmed by thin layer chromatography and reverse phase high performance liquid chromatography.

### Example 2

Preparation of Arg-Val-Thr-His-Pro-His-Leu-Pro-Arg-Ala-Leu-Met-Arg-Ser-Thr-Thr-Lys-Thr-Ser-Gly-Pro-Arg-Ala-Ala-Pro-Glu-Val-Tyr-Ala-Phe-Ala-Thr-Pro-Glu-Trp-Pro-Gly-Ser-Arg-Asp-Lys-Arg-Thr-Leu-Ala (Compound 2):

Fmoc-Ala-resin (i.e. p-alkoxybenzyl alcohol resin introduced with Fmoc-Ala in an amount of 0.41 mmol/g of resin) (731 mg) was transferred to a Merrifield solid phase reaction vessel equipped for agitation, and is suspended in dimethylformamide (20 ml) and shaken for 30 minutes, by which Fmoc-Ala-resin was swollen.

The resultant was subjected to the following Fmoc group-deprotecting cycle.

(a) Shaking in DMF (20 ml) for one minutes, followed by filtering (twice),

(b) Shaking in 20 % piperidine-Dmf solution (20 ml) for three minutes, followed by filtering,

(c) Shaking in 20 % piperidine-DMF solution (20 ml) for 10 minutes, followed by filtering,

(d) Washing twice with DMF (20 ml),

(e) Washing twice with isopropanol (20 ml).

At this stage, it was confirmed by Kaiser method whether Fmoc group was completely deprotected or not. If it was incompleted, the above deprotecting cycle was repeated. If it was completely deprotected, the resultant was subjected to the following synthetic cycle.

(f) Swelling the H-Ala-resin obtained by the above Fmoc group-deprotecting cycle by shaking twice in DMF (20 ml), by which the resin was swollen,

(g) Adding thereto a solution of Fmoc-Leu (334 mg, 0.9 mmole) and HOBt (146 mg, 1.08 mmole) in DMF (20 ml) and shaking the mixture for one minute.

(h) Adding thereto a solution of 1M dicyclohexyl-carbodiimide in methylene chloride (1.36 ml) and shaking the mixture for 70 minutes,

(i) Washing twice with DMF (20 ml),

(j) Washing twice with isopropanol (20 ml).

At this stage, it was confirmed by Kaiser method whether the condensation reaction was completed or not. If it was incompleted, the above condensation cycle was repeated. If it was completed, the reaction product was subjected again to Fmoc group-deprotecting cycle and then to the condensation cycle wherein the step (g) was carried out by using Fmoc-Thr(tBu). Thereafter, by repeating the Fmoc group-deprotecting cycle and Fmoc-amino acid condensation cycle likewise, there were condensed with Fmoc-Arg(Mtr), Fmoc-Lys(Boc), Fmoc-Asp(OtBu), Fmoc-Arg(Mtr), Fmoc-Ser(tBu), Fmoc-Gly, Fmoc-Pro, Fmoc-Trp, Fmoc-Glu-(OtBu), Fmoc-Pro, Fmoc-Thr(tBu), Fmoc-Ala, Fmoc-Phe, Fmoc-Ala, Fmoc-Tyr(tBu), Fmoc-Val, Fmoc-Glu-(OtBu), Fmoc-Pro, Fmoc-Ala, Fmoc-Ala, Fmoc-Arg(Mtr), Fmoc-Pro, Fmoc-Gly, Fmoc-Ser(tBu), Fmoc-Thr-(tBu), Fmoc-Lys(Boc), Fmoc-Thr(tBu), Fmoc-Thr(tBu), Fmoc-Ser(tBu), Fmoc-Arg(Mtr), Fmoc-Met, Fmoc-Leu, Fmoc-Ala, Fmoc-Arg(Mtr), Fmoc-Pro, Fmoc-Leu, Fmoc-His(Fmoc), Fmoc-Pro, Fmoc-His(Fmoc), Fmoc-Thr-(tBu), Fmoc-Val, and Fmoc-Arg(Mtr).

The thus obtained Fmoc-Arg(Mtr)-Val-Thr(tBu)-His(Fmoc)-Pro-His(Fmoc)-Leu-Pro-Arg(Mtr)-Ala-Leu-Met-Arg(Mtr)-Ser(tBu)-Thr(tBu)-Thr(tBu)-Lys(Boc)-Thr(tBu)-Ser(tBu)-Gly-Pro-Arg(Mtr)-Ala-Ala-Pro-Glu(OtBu)-Val-Tyr(tBu)-Ala-Phe-Ala-Thr(tBu)-Pro-Glu(OtBu)-Trp-Pro-Gly-Ser(tBu)-Arg(Mtr)-Asp(OtBu)-Lys(Boc)-Arg(Mtr)-Thr(tBu)-Leu-Ala-resin was subjected to the Fmoc group-removing cycle to give H-Arg(Mtr)-Val-Thr(tBu)-His-Pro-His-Leu-Pro-Arg(Mtr)-Ala-Leu-Met-Arg(Mtr)-Ser(tBu)-Thr(tBu)-Thr(tBu)-Lys(Boc)-Thr(tBu)-Ser(tBu)-Gly-Pro-Arg(Mtr)-Ala-Ala-Pro-Glu(OtBu)-Val-Try(tBu)-Ala-Phe-Ala-Thr(tBu)-Pro-Glu(OtBu)-Trp-Pro-Gly-Ser-(tBu)-Arg(Mtr)-Asp(OtBu)-Lys(Boc)-Arg(Mtr)-Thr(tBu)-Leu-Ala-resin which was then subjected to the cleavage of resin.

That is, the product was washed twice with methylene chloride (20 ml) and suspended in a mixture of methylene chloride (10 ml) - anisole (2.00 ml) - thiophenol (0.66 ml), and thereto was added trifluoroacetic acid (20 ml) - methylene chloride (2.32 ml), and the mixture was shaken for one hour. Afer filtering off the resin, ether was added to the filtrate, and the mixture was filtered to give white precipitate (1.56 g). To the product was added a mixture of trifluoroacetic acid (13.5 ml) - thionisole (1.5 ml) - thiophenol (0.60 ml), and the mixture was stirred for 5 hours. To the reaction mixture was added ether, and the mixture was filtered to give white precipitate (1.50 g). The white powder was subjected to a reverse phase high performance liquid chromatography and there was taken a fraction containing the desired Arg-Val-Thr-His-Pro-His-Leu-Pro-Arg-Ala-Leu-Met-Arg-Ser-Thr-Thr-Lys-Thr-Ser-Gly-Pro-Arg-Ala-Ala-Pro-Glu-Val-Tyr-Ala-Phe-Ala-Thr-Pro-Glu-Trp-Pro-Gly-Ser-Arg-Asp-Lys-Arg-Thr-Leu-Ala, and the eluted fraction was evaporated to dryness. The residue was dissolved in distilled water and then the mixture was concentrated to dryness. This procedure was repeated several times. Thereafter, a small amount of distilled water was added to the residue and the mixture was lyophilized to give purified Arg-Val-Thr-His-Pro-His-Leu-Pro-Arg-Ala-Leu-Met-Arg-Ser-Thr-Thr-Lys-Thr-Ser-Gly-Pro-Arg-Ala-Ala-Pro-Glu-Val-Tyr-Ala-Phe-Ala-Thr-Pro-Glu-Trp-Pro-Gly-Ser-Arg-Asp-Lys-Arg-Thr-Leu-Ala (359 mg).

A part of the purified product was taken and subjected to the amino acid sequence analysis with a gas phase protein sequencer. As a result, it had the sequence of Arg, Val, Thr, His, Pro, His, Leu, Pro, Arg, Ala, Leu, Met, Arg in this order from the N-terminus. Besides, the product was hydrolyzed with 6N HCl and subjected to amino acid analysis. As a result, it showed amino acid ratios of Asp 1.11, Thr 5.68, Ser 3.06, Glu 2.08, Pro 5.92, Gly 2.15, Ala 6.00, Val 1.62, Met 1.02, Leu 2.82, Tyr 0.87, Phe 1.17, Lys 2.12, His 1.60 and Arg 5.63, which were well agreed to the theoretical ratios. Thus, it was confirmed that the desired peptide was obtained.

The high purity of the product was also confirmed by thin layer chromatography and reverse phase high performance liquid chromatography.

## Example 3

Preparation of Pro-Arg-Ala-Leu-Met-Arg-Ser-Thr-Thr-Lys-Thr-Ser-Gly-Pro-Arg (Compound 3):

Fmoc-Arg(Mtr)-resin (i.e. p-alkoxybenzyl alcohol resin introduced with Fmoc-Arg(Mtr) in an amount of 0.39 mmol/g of resin) (769 mg) was transferred to a Merrifield solid phase reaction vessel equipped for agitation, and is suspended in dimethylformamide (20 ml) and shaken for 30 minutes, by which Fmoc-Arg-(Mtr)-resin was swollen.

The resultant was subjected to the following Fmoc group-deprotecting cycle.
(a) Shaking in DMF (20 ml) for one minutes, followed by filtering (twice),
(b) Shaking in 20 % piperidine-DMF solution (20 ml) for three minutes, followed by filtering,
(c) Shaking in 20 % piperidine-DMF solution (20 ml) for 10 minutes, followed by filtering,
(d) Washing twice with DMF (20 ml),
(e) Washing twice with isopropanol (20 ml).

At this stage, it was confirmed by Kaiser method whether Fmoc group was completely deprotected or not. If it was incompleted, the above deprotecting cycle was repeated. If it was completely deprotected, the resultant was subjected to the following synthetic cycle.
(f) Swelling the H-Arg(Mtr)-resin obtained by the above Fmoc group-deprotecting cycle by shaking twice in DMF (20 ml), by which the resin was swollen,
(g) Adding thereto a solution of Fmoc-Pro (309 mg, 0.9 mmole) and HOBt (146 mg, 1.08 mmole) in DMF (20 ml) and shaking the mixture for one minute,
(h) Adding thereto a solution of 1M dicyclohexyl-carbodiimide in methylene chloride (0.99 ml) and shaking the mixture for 70 minutes,
(i) Washing twice with DMF (20 ml),
(j) Washing twice with isopropanol (20 ml).

At this stage, it was confirmed by Kaiser method whether the condensation reaction was completed or not. If it was incompleted, the above condensation cycle was repeated. If it was completed, the reaction product was subjected again to Fmoc group-deprotectding cycle and then to the condensation cycle wherein the step (g) was carried out by using Fmoc-Gly. Thereafter, by repeating the Fmoc groupdeprotecting cycle and Fmoc-amino acid condensation cycle likewise, there were condensed with Fmoc-Ser(tBu), Fmoc-Thr(tBu), Fmoc-Lys(Boc), Fmoc-Thr(tBu), Fmoc-Thr(tBu), Fmoc-Ser(tBu), Fmoc-Arg(Mtr), Fmoc-Met, Fmoc-Leu, Fmoc-Ala, Fmoc-Arg(Mtr), and Boc-Pro.

The thus obtained Boc-Pro-Arg(Mtr)-Ala-Leu-Met-Arg(Mtr)-Ser(tBu)-Thr(tBu)-Thr(tBu)-Lys(Boc)-Thr(tBu)-Ser(tBu)-Gly-Pro-Arg(Mtr)-resin was subjected to the cleavage of resin.

That is, the product was washed twice with methylene chloride (20 ml) and suspended in a mixture of methylene chloride (0 ml) - anisole (2.00 ml) - thiophenol (0.66 ml), and thereto was added trifluoroacetic acid (20 ml) - methylene chloride (2.32 ml), and the mixture was shaken for one hour. After filtering off the resin, ether was added to the filtrate, and the mixture was filtered to give white precipitate (772 mg). To the product was added a mixture of trifluoroacetic acid (13.5 ml) - thioanisole (1.5 ml) - thiophenol (0.60 ml), and the mixture was stirred for 5 hours. To the reaction mixture was added ether, and the mixture was filtered to give white precipitate (550 mg). This product was subjected to a reverse phase high performance liquid chromatography and there was taken a fraction containing the desired Pro-Arg-Ala-Leu-Met-Arg-Ser-Thr-Thr-Lys-Thr-Ser-Gly-Pro-Arg, and the eluted fraction was evaporated to dryness. The residue was dissolved in distilled water and then the mixture was concentrated to dryness. This procedure was repeated several times. Thereafter, a small amount of distilled water was added to the residue and the mixture was lyophilized to give purified Pro-Arg-Ala-Leu-Met-Arg-Ser-Thr-Thr-Lys-Thr-Ser-Gly-Pro-Arg (185 mg).

A part of the purified product was taken and subjected to the amino acid sequence analysis with a gas phase protein sequencer. As a result, it had the sequence of Pro, Arg, Ala, Leu, Met, Arg, Ser, Thr, Thr, Lys, Thr, Ser, Gly, Pro, Arg in this order, which were identical with the peptide sequence. Besides, the product was hydrolyzed with 6N HCl and subjected to amino acid analysis. As a result, it showed amino acid ratios of Pro 2.07, Arg 3.02, Ala 0.97, Leu 1.04, Met 1.01, Ser 1.84, Thr 2.88, Lys 1.03, and Gly 1.02, which were well agreed to the theoretical ratios, too. Thus, it was confirmed that the desired peptide was obtained.

The high purity of the product was also confirmed by thin layer chromatography and reverse phase high performance liquid chromatography.

### Example 4

Preparation of Ser-Arg-Asp-Lys-Arg-Thr (Compound 4):

Fmoc-Thr(tBu)-resin (i.e. p-alkoxybenzyl alcohol resin introduced with Fmoc-Thr(tBu) in an amount of 0.45 mmol/g of resin) (918 mg) was transferred to a Merrifield solid phase reaction vessel equipped for agitation, and is suspended in dimethylformamide (20 ml) and shaken for 30 minutes, by which Fmoc-Thr-(tBu)-resin was swollen.

The resultant was subjected to the following Fmoc group-deprotecting cycle.

(a) Shaking in DMF (20 ml) for one minutes (twice),

(b) Shaking in 20 % piperidine-DMF solution (20 ml) for three minutes,

(c) Shaking in 20 % piperidine-DMF solution (20 ml) for 10 minutes to deprotect Fmoc group,

(d) Washing twice with DMF (20 ml),

(e) Washing twice with isopropanol (20 ml).

At this stage, it was confirmed by Kaiser method whether Fmoc group was completely deprotected or not. If it was incompleted, the above deprotecting cycle was repeated. If it was completely deprotected, the resultant was subjected to the following synthetic cycle.

(f) Swelling the H-Thr(tBu)-resin obtained by the above Fmoc group-deprotecting cycle by shaking twice in DMF (20 ml), by which the resin was swollen,

(g) Adding thereto a solution of Fmoc-Arg(Mtr) (756 mg, 1.24 mmole) and HOBt (200 mg, 1.48 mmole) in DMF (20 ml) and shaking the mixture for one minute,

(h) Adding thereto a solution of 1M dicyclohexyl-carbodiimide in methylene chloride (1.36 ml) and shaking the mixture for 70 minutes,

(i) Washing twice with DMF (20 ml),

(j) Washing twice with isopropanol (20 ml).

At this stage, it was confirmed by Kaiser method whether the condensation reaction was completed or not. If it was incompleted, the above condensation cycle was repeated. If it was completed, the reaction product was subjected again to Fmoc group-deprotecting cycle and then to the condensation cycle wherein the step (g) was carried out by using Fmoc-Lys(Boc). Thereafter, by repeating the Fmoc group-deprotecting cycle and Fmoc-amino acid condensation cycle likewise, there were condensed with Fmoc-Asp(OtBu), Fmoc-Arg(Mtr), and Fmoc-Ser(tBu).

The thus obtained Fmoc-Ser(tBu)-Arg(Mtr)-Asp(OtBu)-Lys(Boc)-Arg(Mtr)-Thr(tBu)-resin was subjected to the Fmoc group-deprotecting cycle to give H-Ser(tBu)-Arg(Mtr)-Asp(OtBu)-Lys(Boc)-Arg(Mtr)-Thr(tBu)-resin, which was subjected to the cleavage of resin.

That is, the product was washed twice with methylene chloride (20 ml) and suspended in a mixture of methylene chloride (10 ml) - anisole (2.00 ml) - thiophenol (0.66 ml), and thereto was added trifluoroacetic acid (20 ml) - methylene chloride (2.32 ml), and the mixture was shaken for one hour. After filtering off the resin, ether was added to the filtrate, and the mixture was filtered to give white precipitate (650 mg). To the product was added a mixture of trifluoroacetic acid (13.5 ml) - thioanisole (1.5 ml) - thiophenol (0.60 ml), and the mixture was stirred for 5 hours. To the reaction mixture was added ether, and the mixture was filtered to give white precipitate (360 mg). This product was pulverized with $H_2O$ - ethanol - ether and thereby thioanisole and thiophenol were removed. The resulting white powder (320 mg) was subjected to a reverse phase high performance liquid chromatography and there was taken a fraction containing the desired Ser-Arg-Asp-Lys-Arg-Thr, and the eluted fraction was concentrated to dryness. The residue was dissolved in distilled water and then the mixture was evaporated to dryness. This procedure was repeated several times. Thereafter, a small amount of distilled water was added to the residue and the mixture was lyophilized to give purified Ser-Arg-Asp-Lys-Arg-Thr (139 mg).

A part of the purified product was taken and subjected to the amino acid sequence analysis with a gas phase protein sequencer. As a result, it had the sequence of Ser, Arg, Asp, Lys, Arg, Thr in this order, which were identical with the desired amino acid sequnce. Besides, the molecular weight of the product was measured with an FAB mass spectrometer, and the obtained data: m/z 762 (M$^+$ + H) were well agreed to the theoretical amount. Moreover, the product was hydrolyzed with 6N HCl and subjected to amino acid analysis. As a result, it showed amino acid ratios of Ser 0.91, Arg 2.02, Asp 1.00, Lys 1.00, Tyr 0.97, which were well agreed to the theoretical ratios, too. Thus, it was confirmed that the desired peptide was obtained.

The high purity of the product was also confirmed by thin layer chromatography and reverse phase high performance liquid chromatography.

## Example 5

Preparation of Ala-Asp-Ser-Asn-Pro-Arg-Gly-Val-Ser-Ala-Tyr-Leu-Ser-Arg-Pro-Ser-Pro-Phe-Asp-Leu (Compound 5):

In the same manner as described in Examples 1 to 4 using Fmoc-Leu-resin (i.e. p-alkoxybenzyl alcohol resin introduced with Fmoc-Leu in an amount of 0.48 mmol/g of resin) (960 mg) as the starting material, there was prepared purified Ala-Asp-Ser-Asn-Pro-Arg-Gly-Val-Ser-Ala-Tyr-Leu-Ser-Arg-Pro-Ser-Pro-Phe-Asp-Leu (483 mg).

A part of the purified product was taken and subjected to the amino acid sequence analysis with a gas phase protein sequencer. As a result, it had the sequence of Ala, Asp, Ser, Asn, Pro, Arg, Gly, Val, Ser, Ala, Tyr, Leu, Se, Arg, Pro, Ser, Pro, Phe, Asp, Leu in this order, which were identical with the peptide sequence. Besides, the molecular weight of the product was measured with an FAB mass spectrometer, and the obtained data: m/z 2149 (M$^+$ + H) were well agreed to the theoretical amount. Moreover, the product was hydrolyzed with 6N HCl and subjected to amino acid analysis. As a result, it showed amino acid ratios of Ala 1.89, Asp 2.75, Ser 3.63, Pro 3.35, Arg 1.98, Gly 0.96, Val 0.95, Leu 2.25, Tyr 0.77, and Phe 1.16, which were well agreed to the theoretical ratios, too. Thus, it was confirmed that the desired peptide was obtained.

The high purity of the product was also confirmed by thin layer chromatography and reverse phase high performance liquid chromatography.

## Example 6

Preparation of Pro-Phe-Asp-Leu-Phe-Ile-Arg-Lys-Ser-Pro-Thr-Ile (Compound 6):

In the same manner as described in Examples 1 to 4 using Fmoc-Ile-resin (i.e. p-alkoxybenzyl alcohol resin introduced with Fmoc-Ile in an amount of 0.57 mmol/g of resin) (526 mg) as the starting material, there was prepared purified Pro-Phe-Asp-Leu-Phe-Ile-Arg-Lys-Sr-Pro-Thr-Ile (202 mg).

A part of the purified product was taken and subjected to the amino acid sequence analysis with a gas phase protein sequencer. As a result, it had the sequence of Pro, Phe, Asp, Leu, Phe, Ile, Arg, Lys, Ser, Pro, Thr, Ile, in this order, which were identical with the desired amino acid sequence. Besides, the product was hydrolyzed with 6N HCl and subjected to amino acid analysis. As a result, it showed amino acid ratios of Asp 0.98, Thr 0.93, Ser 0.93, Ile 1.18, Leu 1.02, Phe 1.99, Lys 1.01, Arg 0.94, and Pro 2.01, which were well agreed to the theoretical ratios, too. Thus, it was confirmed that the desired peptide was obtained.

The high purity of the product was also confirmed by thin layer chromatography and reverse phase high performance liquid chromatography.

Example 7

Preparation of Gln-Ser-Arg-Val-Thr-His-Pro-His-Leu-Pro-Arg-Ala-Leu-Met-Arg-Ser-Thr-Thr-Lys-Thr-Ser (Compound 7):

In the same manner as described in Examples 1 to 4 using Fmoc-Ser(tBu)-resin (i.e. p-alkoxybenzyl alcohol resin introduced with Fmoc-Ser(tBu) in an amount of 0.40 mmol/g of resin) (500 mg) as the starting material, there was prepared purified Gln-Ser-Arg-Val-Thr-His-Pro-His-Leu-Pro-Arg-Ala-Leu-Met-Arg-Ser-Thr-Thr-Lys-Thr-Ser (250 mg).

A part of the purified product was taken and subjected to the amino acid sequence analysis with a gas phase protein sequencer. As a result, it had the sequence of Gln, Ser, Arg, Val, Thr, His, Pro, His, Leu, Pro, Arg, Ala, Leu, Met, Arg, Ser, Thr, Thr, Lys, Thr, Ser in this order, which were identical with the amino acid sequence. Besides, the product was hydrolyzed with 6N HCl and subjected to amino acid analysis. As a result, it showed amino acid ratios of Glu 0.98, Ala 0.98, Thr 3.76, Ser 2.79, Val 0.96, Met 0.75, Leu 2.04, Lys 1.07, His 1.82, Arg 2.96, and Pro 1.82, which were well agreed to the theoretical ratios, too. Thus, it was confirmed that the desired peptide was obtained.

The high purity of the product was also confirmed by thin layer chromatography and reverse phase high performance liquid chromatography.

Example 8

Preparation of Pro-Arg-Ala-Leu-Met-Arg-Ser-Thr (Compound 8):

In the same manner as described in Examples 1 to 4 using Fmoc-Thr(tBu)-resin (i.e. p-alkoxybenzyl alcohol resin introduced with Fmoc-Thr(tBu) in an amount of 0.69 mmol/g of resin) (435 mg) as the starting material, there was prepared purified Pro-Arg-Ala-Leu-Met-Arg-Ser-Thr (93 mg).

A part of the purified product was taken and subjected to the amino acid sequence analysis with a gas phase protein sequencer. As a result, it had the sequence of Pro, Arg, Ala, Leu, Met, Arg, Ser, Thr in this order, which were identical with the peptide sequence. Besides, the molecular weight of the product was measured with an FAB mass spectrometer, and the obtained data: m/z 931 (MH$^+$) were well agreed to the theoretical amount. Moreover, the product was hydrolyzed with 6N HCl and subjected to amino acid analysis. As a result, it showed amino acid ratios of Pro 0.98, Arg 1.97, Ala 0.96, Leu 1.04, Met 0.99, Ser 0.89, and Thr 1.00, which were well agreed to the theoretical ratios, too. Thus, it was confirmed that the desired peptide was obtained.

The high purity of the product was also confirmed by thin layer chromatography and reverse phase high performance liquid chromatography.

Example 9

Preparation of Gly-Pro-Arg-Ala-Ala-Pro-Glu-Val-Tyr-Ala-Phe-Ala-Thr-Pro-Glu-Trp-Pro-Gly-Ser (Compound 9):

In the same manner as described in Examples 1 to 4 using Fmoc-Ser(tBu)-resin (i.e. p-alkoxybenzyl alcohol resin introduced with Fmoc-Ser(tBu) in an amount of 0.54 mmol/g of resin) (927 mg) as the starting material, there was prepared purified Gly-Pro-Arg-Ala-Ala-Pro-Glu-Val-Tyr-Ala-Phe-Ala-Thr-Pro-Glu-Trp-Pro-Gly-Ser (563 mg).

A part of the purified product was taken and subjected to the amino acid sequence analysis with a gas phase protein sequencer. As a result, it had the sequence of Gly, Pro, Arg, Ala, Ala, Pro, Glu, Val, Tyr, Ala, Phe, Ala, Thr, Pro, Glu, Trp, Pro, Gly, Ser in this order, which were identical with the amino acid sequence. Besides, the molecular weight of the product was measured with an FAB mass spectrometer, and the obtained data: m/z 2003 ($M^+ + H$) were well agreed to the theoretical amount. Moreover, the product was hydrolyzed with 6N HCl and subjected to amino acid analysis. As a result, it showed amino acid ratios of Thr 0.97, Ser 0.95, Glu 2.00, Gly 2.04, Ala 4.00, Val 1.00, Tyr 0.98, Phe 1.10, Arg 0.98, Trp 0.21, and Pro 4.09, which were well agreed to the theoretical ratios, too. Thus, it was confirmed that the desired peptide was obtained.

The high purity of the product was also confirmed by thin layer chromatography and reverse phase high performance liquid chromatography.

## Example 10

Preparation of Pro-Gly-Ser-Arg-Asp-Lys-Arg-Thr-Leu-Ala-Ser-Leu-Ile (Compound 10):

In the same manner as described in Examples 1 to 4 using Fmoc-Ile-resin (i.e. p-alkoxybenzyl alcohol resin introduced with Fmoc-Ile in an amount of 0.57 mmol/g of resin) (950 mg) as the starting material, there was prepared purified Pro-Gly-Ser-Arg-Asp-Lys-Arg-Thr-Leu-Ala-Ser-Leu-Ile (420 mg).

A part of the purified product was taken and subjected to the amino acid sequence analysis with a gas phase protein sequencer. As a result, it had the sequence of Pro, Gly, Ser, Arg, Asp, Lys, Arg, Thr, Leu, Ala, Ser, Leu, Ile in this order, which were identical with the peptide sequence. Besides, the molecular weight of the product was measured with an FAB mass spectrometer, and the obtained data: m/z 1413 ($M^+ + H$) were well agreed to the theoretical amount. Moreover, the product was hydrolyzed with 6N HCl and subjected to amino acid analysis. As a result, it showed amino acid ratios of Asp 0.98, Thr 0.97, Ser 1.80, Pro 0.97, Gly 1.00, Ala 1.02, Ile 1.01, Leu 2.00, Lys 1.00, and Arg 1.78, which were well agreed to the theoretical ratios, too. Thus, it was confirmed that the desired peptide was obtained.

The high purity of the product was also confirmed by thin layer chromatography and reverse phase high performance liquid chromatography.

## Example 11

Preparation of Gly-Ser-Arg-Asp-Lys-Arg-Thr (Compound 11):

In the same manner as described in Examples 1 to 4 using Fmoc-Thr(tBu)-resin (i.e. p-alkoxybenzyl alcohol resin introduced with Fmoc-Thr(tBu) in an amount of 0.69 mmol/g of resin) (435 mg) as the starting material, there was prepared purified Gly-Ser-Arg-Asp-Lys-Arg-Thr (107 mg).

A part of the purified product was taken and subjected to the amino acid sequence analysis with a gas phase protein sequencer. As a result, it had the sequence of Gly, Ser, Arg, Asp, Lys, Arg, Thr in this order, which were identical with the peptide sequence. Besides, the molecular weight of the product was measured with an FAB mass spectrometer, and the obtained data: m/z 819 ($MH^+$) were well agreed to the theoretical amount. Moreover, the product was hydrolyzed with 6N HCl and subjected to amino acid analysis. As a result, it showed amino acid ratios of Gly 0.92, Ser 0.92, Arg 2.00, Asp 1.00, Lys 1.01, and Thr 0.98, which were well agreed to the theoretical ratios, too. Thus, it was confirmed that the desired peptide was obtained.

The high purity of the product was also confirmed by thin layer chromatography and reverse phase high performance liquid chromatography.

## Example 12

Preparation of Ser-Arg-Asp-Lys-Arg-Thr-Leu-Ala-Ser-Leu-Ile-Gln-Asn-Phe-Met (Compound 12):

In the same manner as described in Examples 1 to 4 using Fmoc-Met-resin (i.e. p-alkoxybenzyl alcohol resin introduced with Fmoc-Met in an amount of 0.43 mmol/g of resin) (697 mg) as the starting material, there was prepared purified Ser-Arg-Asp-Lys-Arg-Thr-Leu-Ala-Ser-Leu-Ile-Gln-Asn-Phe-Met (82 mg).

A part of the purified product was taken and subjected to the amino acid sequence analysis with a gas phase protein sequencer. As a result, it had the sequence of Ser, Arg, Asp, Lys, Arg, Thr, Leu, Ala, Ser, Leu, Ile, Gln, Asn, Phe, Met in this order, which were identical with the peptide sequence. Moreover, the product was hydrolyzed with 6N HCl and subjected to amino acid analysis. As a result, it showed amino acid ratios of Asp 1.98, Thr 0.98, Ser 1.82, Glu 0.96, Ala 0.98, Met 1.07, Ile 1.00, Leu 2.07, Phe 1.09, Lys 1.01, and Arg 1.88, which were well agreed to the theoretical ratios, too. Thus, it was confirmed that the desired peptide was obtained.

The high purity of the product was also confirmed by thin layer chromatography and reverse phase high performance liquid chromatography.

Reference Example

In the same manner as described in Examples 1 to 4 by using a corresponding starting material, the following compounds were prepared.

Reference Compound 13:

Tyr-Leu-Ser-Arg-Pro-Ser-Pro-Phe-Asp-Leu

Reference Compound 14:

Ser-Thr-Thr-Lys-Thr-Ser-Gly

**Claims**

CLAIMS FOR THE FOLLOWING CONTRACTING STATES: GB,FR,DE,IT.

1. A peptide which is (1) selected from the group consisting of a peptide having 1st to 20th amino acid sequence, a peptide having 1st to 29th amino acid sequence, and a peptide having 17th to 28th amino acid sequence in the formula [I]:

$$\underset{1}{\text{Ala}}\text{-Asp-Ser-Asn-Pro-Arg-Gly-Val-Ser-}\underset{10}{\text{Ala}}\quad -$$

$$\text{Tyr-Leu-Ser-Arg-Pro-Ser-Pro-Phe-Asp-}\underset{20}{\text{Leu}}\quad -$$

$$\text{Phe-Ile-Arg-Lys-Ser-Pro-Thr-Ile-}\underset{29}{\text{Thr}}$$

or (2) selected from the group consisting of a peptide having 1st to 21st amino acid sequence, a peptide having 3rd to 47th amino acid sequence, a peptide having 10th to 17th amino acid sequence, a peptide having 10th to 24th amino acid sequence, a peptide having 22nd to 40th amino acid sequence, a peptide having 38th to 50th amino acid sequence, a peptide having 39th to 45th amino acid sequence, a peptide having 40th to 45th amino acid sequence, and a peptide having 40th to 54th amino acid sequence in the formula [II]:

$$\underset{1}{\text{Gln}}\text{-X -Arg-Val-Thr-His-Pro-His-Leu-}\underset{10}{\text{Pro}}\quad -$$

$$\text{Arg-Ala-Leu-Met-Arg-Ser-Thr-Thr-Lys-}\underset{20}{\text{Thr}}\quad -$$

$$\text{Ser-Gly-Pro-Arg-Ala-Ala-Pro-Glu-Val-}\underset{30}{\text{Tyr}}\quad -$$

$$\text{Ala-Phe-Ala-Thr-Pro-Glu-Trp-Pro-Gly-}\underset{40}{\text{Ser}}\quad -$$

$$\text{Arg-Asp-Lys-Arg-Thr-Leu-Ala-X -Leu-}\underset{50}{\text{Ile}}\quad -$$

$$\text{Gln-Asn-Phe-}\underset{54}{\text{Met}}$$

wherein X is Cys or Ser,

or (3) a partially modified peptide at an amino acid residue thereof, or (4) a salt of any of the peptides (1), (2) and (3).

2. The peptide according to claim 1, which is a member selected from the group consisting of a peptide having 1st to 20th amino acid sequence, a peptide having 1st to 29th amino acid sequence, and a peptide having 17th to 28th amino acid sequence in the formula [I].

3. The peptide according to claim 1, which is a member selected from the group consisting of a peptide having 1st to 21st amino acid sequence, a peptide having 3rd to 47th amino acid sequence, a peptide having 10th to 17th amino acid sequence, a peptide having 10th to 24th amino acid sequence, a peptide having 22nd to 40th amino acid sequence, a peptide having 38th to 50th amino acid sequence, a peptide having 39th to 45th amino acid sequence, a peptide having 40th to 45th amino acid sequence, and a peptide having 40th to 54th amino acid sequence in the formula [II].

4. The peptide according to claim 2 which is a peptide having an amino acid sequence of the formula [III]:

Ala-Asp-Ser-Asn-Pro-Arg-Gly-Val-Ser-Ala-Tyr-Leu-Ser-Arg-Pro-Ser-Pro-Phe-Asp-Leu.

5. The peptide according to claim 2 which is a peptide having an amino acid sequence of the formula [IV]:

Ala-Asp-Ser-Asn-Pro-Arg-Gly-Val-Ser-Ala-Tyr-Leu-Ser-Arg-Pro-Ser-Phe-Asp-Leu-Phe-Ile-Arg-Lys-Ser-Pro-Thr-Ile-Thr.

6. The peptide according to claim 2 which is a peptide having an amino acid sequence of the formula [V]:

Pro-Phe-Asp-Leu-Phe-Ile-Arg-Lys-Ser-Pro-Thr-Ile.

7. The peptide according to claim 3 which is a peptide having an amino acid sequence of the formula [VI]:

Gln-Ser-Arg-Val-Thr-His-Pro-His-Leu-Pro-Arg-Ala-Leu-Met-Arg-Ser-Thr-Thr-Lys-Thr-Ser.

8. The peptide according to claim 3 which is a peptide having an amino acid sequence of the formula [VII]:

Arg-Val-Thr-His-Pro-His-Leu-Pro-Arg-Ala-Leu-Met-Arg-Ser-Thr-Thr-Lys-Thr-Ser-Gly-Pro-Arg-Ala-Ala-Pro-Glu-Val-Tyr-Ala-Phe-Ala-Thr-Pro-Glu-Trp-Pro-Gly-Ser-Arg-Asp-Lys-Arg-Thr-Leu-Ala.

9. The peptide according to claim 3 which is a peptide having an amino acid sequence of the formula [VIII]:

Pro-Arg-Ala-Leu-Met-Arg-Ser-Thr.

10. The peptide according to claim 3 which is a peptide having an amino acid sequence of the formula [IX]:

Pro-Arg-Ala-Leu-Met-Arg-Ser-Thr-Thr-Lys-Thr-Ser-Gly-Pro-Arg.

11. The peptide according to claim 3 which is a peptide having an amino acid sequence of the formula [X]:

Gly-Pro-Arg-Ala-Ala-Pro-Glu-Val-Tyr-Ala-Phe-Ala-Thr-Pro-Glu-Trp-Pro-Gly-Ser.

12. The peptide according to claim 3 which is a peptide having an amino acid sequence of the formula [XI]:

Pro-Gly-Ser-Arg-Asp-Lys-Arg-Thr-Leu-Ala-Ser-Leu-Ile.

13. The peptide according to claim 3 which is a peptide having an amino acid sequence of the formula [XII]:

Gly-Ser-Arg-Asp-Lys-Arg-Thr.

14. The peptide according to claim 3 which is a peptide having an amino acid sequence of the formula [XIII]:

Ser-Arg-Asp-Lys-Arg-Thr.

15. The peptide according to claim 3 which is a peptide having an amino acid sequence of the formula [XIV]:

Ser-Arg-Asp-Lys-Arg-Thr-Leu-Ala-Ser-Leu-Ile-Gln-Asn-Phe-Met.

16. The peptide according to claim 1 which is in the form of a pharmaceutically acceptable salt.

17. A peptide as set forth in claim 1 or a pharmaceutically acceptable salt thereof for use in prophylaxis and treatment of allergic diseases.


CLAIMS FOR THE FOLLOWING CONTRACTING STATE: ES

1. A method for preparing a peptide wherein the final product is a peptide which is (1) selected from the group consisting of a peptide having 1st to 20th amino acid sequence, a peptide having 1st to 29th amino acid sequence, and a peptide having 17th to 28th amino acid sequence in the formula [I]:

A l a-Asp-Ser-Asn-Pro-Arg-Gly-Val-Ser-Ala    -
1                                                          10

Tyr-Leu-Ser-Arg-Pro-Ser-Pro-Phe-Asp-Leu -
20

Phe-Ile-Arg-Lys-Ser-Pro-Thr-Ile-Thr
29

or (2) selected from the group consisting of a peptide having 1st to 21st amino acid sequence, a peptide having 3rd to 47th amino acid sequence, a peptide having 10th to 17th amino acid sequence, a peptide having 10th to 24th amino acid sequence, a peptide having 22nd to 40th amino acid sequence, a peptide having 38th to 50th amino acid sequence, a peptide having 39th to 45th amino acid sequence, a peptide having 40th to 45th amino acid sequence, and a peptide having 40th to 54th amino acid sequence in the formula [II]:

Gl n-X -Arg-Val-Thr-His-Pro-His-Leu-Pro -
1                                  10

Arg-Ala-Leu-Met-Arg-Ser-Thr-Thr-Lys-Thr -
20

Ser-Gly-Pro-Arg-Ala-Ala-Pro-Glu-Val-Tyr -
30

Ala-Phe-Ala-Thr-Pro-Glu-Trp-Pro-Gly-Ser -
40

Arg-Asp-Lys-Arg-Thr-Leu-Ala-X -Leu-Ile -
50

Gln-Asn-Phe-Met
54

wherein X is Cys or Ser,
or (3) a partially modified peptide at an amino acid residue threof, or (4) a salt of any of the peptides (1), (2) and (3).

2. A method according to claim 1, wherein the peptide is a member selected from the group consisting of a peptide having 1st to 20th amino acid sequence, a peptide having 1st to 29th amino acid sequence, and a peptide having 17th to 28th amino acid sequence in the formula [I].

3. A method according to claim 1, wherein the peptide is a member selected from the group consisting of a peptide having 1st to 21st amino acid sequence, a peptide having 3rd to 47th amino acid sequence, a peptide having 10th to 17th amino acid sequence, a peptide having 10th to 24th amino acid sequence, a peptide having 22nd to 40th amino acid sequence, a peptide having 38th to 50th amino acid sequence, a peptide having 39th to 45th amino acid sequence, a peptide having 40th to 45th amino acid sequence, and a peptide having 40th to 54th amino acid sequence in the formula [II].

4. A method according to claim 2, wherein the peptide has an amino acid sequence of the formula [III]:
Ala-Asp-Ser-Asn-Pro-Arg-Gly-Val-Ser-Ala-Tyr-Leu-Ser-Arg-Pro-Ser-Pro-Phe-Asp-Leu.

5. A method according to claim 2, wherein the peptide has an amino acid sequence of the formula [IV]:
Ala-Asp-Ser-Asn-Pro-Arg-Gly-Val-Ser-Ala-Tyr-Leu-Ser-Arg-Pro-Ser-Pro-Phe-Asp-Leu-Phe-Ile-Arg-Lys-Ser-Pro-Thr-Ile-Thr.

6. A method according to claim 2, wherein the peptide has an amino acid sequence of the formula [V]:
Pro-Phe-Asp-Leu-Phe-Ile-Arg-Lys-Ser-Pro-Thr-Ile.

7. A method according to claim 3, wherein the peptide has an amino acid sequence of the formula [IV]:
Gln-Ser-Arg-Val-Thr-His-Pro-His-Leu-Pro-Arg-Ala-Leu-Met-Arg-Ser-Thr-Thr-Lys-Thr-Ser.

8. A method according to claim 3, wherein the peptide has an amino acid sequence of the formula [VII]:
Arg-Val-Thr-His-Pro-His-Leu-Pro-Arg-Ala-Leu-Met-Arg-Ser-Thr-Thr-Lys-Thr-Ser-Gly-Pro-Arg-Ala-Ala-Pro-Glu-Val-Tyr-Ala-Phe-Ala-Thr-Pro-Glu-Trp-Pro-Gly-Ser-Arg-Asp-Lys-Arg-Thr-Leu-Ala.

9. A method according to claim 3, wherein the peptide has an amino acid sequence of the formula [VIII]:
Pro-Arg-Ala-Leu-Met-Arg-Ser-Thr.

10. A method according to claim 3, wherein the peptide has an amino acid sequence of the formula [IX]:
Pro-Arg-Ala-Leu-Met-Arg-Ser-Thr-Thr-Lys-Thr-Ser-Gly-Pro-Arg.

11. A method according to claim 3, wherein the peptide has an amino acid sequence of the formula [X]:
Gly-Pro-Arg-Ala-Ala-Pro-Glu-Val-Tyr-Ala-Phe-Ala-Thr-Pro-Glu-Trp-Pro-Gly-Ser.

12. A method according to claim 3, wherein the peptide has an amino acid sequence of the formula [XI]:
Pro-Gly-Ser-Arg-Asp-Lys-Arg-Thr-Leu-Ala-Ser-Leu-Ile.

16

13. A method according to claim 3, wherein the peptide has an amino acid sequence of the formula [XII]:

Gly-Ser-Arg-Asp-Lys-Arg-Thr.

14. A method according to claim 3, wherein the peptide has an amino acid sequence of the formula [XIII]:

Ser-Arg-Asp-Lys-Arg-Thr.

15. A method according to claim 3, wherein the peptide has an amino acid sequence of the formula [XIV]:

Ser-Arg-Asp-Lys-Arg-Thr-Leu-Ala-Ser-Leu-Ile-Gln-Asn-Phe-Met.

16. A method according to claim 1 which includes salifying the resultant product to form a pharmaceutically acceptable salt.